# FASCICULE DE BREVET EUROPEEN

(11) **EP 1 066 829 B1**
(45) Date de publication et mention de la délivrance du brevet: **26.03.2003**
(21) Numéro de dépôt: 00401902.2
(22) Date de dépôt: 03.07.2000
(51) Int. Cl.: A61K 9/107, A61K 9/72, A61K 31/20

(54) **Microémulsions stables pour l'administration d'acides gras, et utilisation de ces microémulsions**
Stabile Mikroemulsionen zur Verabreichung von Fettsäuren, und deren Verwendung
Stable microemulsions for administration of fatty acids, and use thereof

(30) Priorité: 05.07.1999 FR 9908653
(43) Date de publication de la demande: 10.01.2001
(73) Titulaire: Ceva Sante Animale, 33501 Libourne Cedex (FR)
(72) Inventeur: Zanello, Philippe, 33130 Begles (FR)
(74) Mandataire: Bernasconi, Jean Raymond

(56) Documents cités:
- EP-A- 0 526 666
- EP-A- 0 608 828

## Description

La présente invention concerne des microémulsions stables pour l'administration d'acides gras à l'homme ou à l'animal. Ces microémulsions d'un pH inférieur à 6,5 sont fortement concentrées en acide gras puisqu'elles contiennent de 5 à 30% en poids d'acides gras.

L'influence des acides gras et de leurs mélanges sur le comportement animal a été décrite et commentée dans la littérature. A titre d'illustration, on peut citer les phéromones qui peuvent contenir ou constituer de tels mélanges d'acides gras.

On connaît plus particulièrement des compositions à base d'acides gras ayant un effet apaisant et relaxant sur certains mammifères. Ces compositions sont capables de combattre l'anxiété d'un animal vivant une situation de stress particulière, occasionnée par exemple par un changement trop brusque de son environnement. U.S. 5 709 863 décrit notamment des compositions d'acides gras qui, par simple mise en contact avec les organes olfactifs des voies respiratoires, facilitent l'adaptation du chat à un environnement inconnu en limitant son anxiété.

Les émulsions décrites dans US 5 709 863 présentent cependant un certain nombre d'inconvénients.

Elles ne sont pas facilement dispersables dans l'environnement du fait de leur viscosité importante.

De plus, elles présentent une thermostabilité insuffisante : on a pu observer une cristallisation des acides gras à basse température et parfois même à température ambiante.

Une façon de réduire la viscosité des compositions d'acides gras est de formuler ces acides gras dans une microémulsion.

Cependant, la formulation de microémulsions concentrées en acides gras qui soient stables à la fois chimiquement et thermiquement est problématique.

De fait, en raison des concentrations élevées en acides gras (supérieures à 5% en poids), l'utilisation de cotensioactifs tels que des alcools est nécessaire. Toutefois, la présence simultanée d'alcools et d'acides gras dans les microémulsions n'est a priori pas souhaitable étant donné les risques importants d'estérification. La stabilité chimique de telles microémulsions semble compromise.

De façon étonnante, les inventeurs ont pu mettre au point une composition stable, sous forme de microémulsion, comprenant une concentration élevée en acides gras et pouvant inclure jusqu'à 30% en poids d'alcool.

Plus précisément, l'invention a trait à une composition non détergente, stable, sous forme de microémulsion, pour l'administration à l'homme ou à l'animal, comprenant :
- de 5 à 30% en poids d'un ou plusieurs acides gras à fonction acide carboxylique libre, à titre de principe actif ;
- de 5 à 35% en poids d'un ou plusieurs tensioactifs ;
- de 5 à 30% en poids d'un ou plusieurs alcools en C₁-C₁₂, à titre de cotensioactif ;
- de 5 à 35% en poids d'un ou plusieurs composés hydrosolubles à fonction carbonyle ou hydroxyle ; et
- de 0 à 35% en poids d'un ou plusieurs composés immiscibles à l'eau ;
ladite composition présentant un pH inférieur à 6,5.

Par acide gras, on entend, selon l'invention, des acides monocarboxyliques à chaîne hydrocarbonée, saturés ou insaturés, linéaires ou ramifiés, actifs vis-à-vis de l'homme ou de l'animal lorsque administrés à celui-ci, et ceci quelle que soit la voie d'administration. Plus précisément, ces acides gras sont des substances chimiques susceptibles de modifier le comportement ou les réponses physiologiques de l'homme ou de l'animal.

Habituellement, les acides gras sont en C₄-C₂₂. Des exemples en sont les acides caprique, laurique, myristique, palmitique, palmitoléique, oléique, linoléique, stéarique, arachidonique, n-butyrique, isobutyrique, α-méthylbutyrique, caproïque, pivalique, gammalinoléique, eicosapentanoïque et docosahexanoïque.

A température ambiante, ces acides gras se présentent soit sous forme liquide, soit sous forme solide, en fonction de la longueur et de la structure de la chaîne carbonée.

Des exemples de mélanges d'acides gras utilisables comme principe actif dans le cadre de l'invention sont les phéromones.

De manière préférée, les compositions de l'invention comprennnent un des mélanges d'acide gras suivants :
i - un mélange d'acide oléique et d'acide palmitique ;
ii - un mélange d'acide oléique et d'acide n-butyrique ;
iii - un mélange d'acide oléique, d'acide palmitique et d'acide linoléique ;
iv - un mélange d'acide oléique, d'acide palmitique, d'acide linoléique et d'acide palmitoléique ;
v - un mélange d'acide caprique, d'acide laurique, d'acide myristique, d'acide palmitoléique, d'acide palmitique, d'acide linoléique et d'acide oléique ;
vi - un mélange d'acide oléique, d'acide palmitique, d'acide linoléique et d'acide myristique ;
vii - un mélange d'acide oléique, d'acide palmitique, d'acide linoléique, d'acide laurique et d'acide myristique ;
viii - un mélange d'acide oléique, d'acide palmitique, d'acide linoléique, d'acide myristique et d'acide pentadécanoïque ;
ix - un mélange d'acide oléique, d'acide palmitique, d'acide linoléique, d'acide myristique, d'acide pentadécanoïque et d'acide stéarique ;
x - un mélange d'acide oléique, d'acide palmitique, d'acide linoléique, d'acide myristique, d'acide laurique et d'acide pentadécanoïque ;
xi - un des mélanges d'acide gras en C₄-C₂₂ décrits dans U.S. 5 709 863 ;

La composition de l'invention comprend préférablement de 10 à 30% en poids d'un ou plusieurs acides gras, mieux encore de 15 à 25% en poids, par exemple de 17 à 23% en poids.

Selon l'invention, il est essentiel que le pH de la composition soit inférieur à 6,5. De préférence, le pH de cette composition est compris entre 3 et 6.

Selon l'invention, la composition sous forme de microémulsion peut comprendre un ou plusieurs tensioactifs choisis parmi un tensioactif anionique, cationique, zwitterionique ou non-ionique dès lors que sa nature n'est pas incompatible avec son utilisation.

De préférence, les compositions de l'invention comprennent exclusivement des constituants pharmaceutiquement acceptables.

Ainsi, les tensioactifs préférés sont les tensioactifs anioniques et mieux encore non-ioniques.

Des exemples de tensioactifs anioniques pouvant convenir sont les alkylsulfates dans lesquels la chaîne alkyle est en C₆-C₁₈ tels que le laurylsulfate de sodium, des alkylbénzènesulfonates dans lesquels la chaîne alkyle est en C₆-C₁₈ et les dialkylsulfosuccinates dans lesquels la chaîne alkyle est en C₆-C₁₈ tel que le dioctylsulfosuccinate de sodium

Le contre-ion des alkylsulfates, des alkylbenzènesulfonates et des dialkylsulfosuccinates est de préférence le cation d'un métal alcalin tel que le sodium ou le potassium, ou bien un cation ammonium.

Des exemples de tensioactifs non ioniques sont :
a) le produit de condensation d'un alcool gras aliphatique, de préférence en C₈-C₂₂, avec un oxyde d'alkylène en C₂-C₃. L'oxyde d'alkylène en C₂-C₃ peut être l'oxyde d'éthylène, l'oxyde de propylène, ou bien un mélange d'oxyde d'éthylène et d'oxyde de propylène dans des proportions quelconques. Un exemple de tels tensioactifs est le produit de condensation de l'alcool laurylique (ou alcool n-dodécyclique) avec 30 moles d'oxyde d'éthylène ;
b) le produit de condensation d'un alkylphénol dans lequel la chaîne alkyle est en C₈-C₂₂ avec un oxyde d'alkylène en C₂-C₃. Là encore, les produits de condensation avec l'oxyde d'éthylène, l'oxyde de propylène ou bien un mélange d'oxyde d'éthylène et d'oxyde de propylène dans des proportions quelconques sont également avantageux. A titre d'exemple de tels tensioactifs, on peut citer le produit de condensation du n-nonylphénol avec 10 moles d'oxyde d'éthylène ;
c) le produit de condensation d'un acide gras de préférence en C₈-C₂₂ avec un oxyde d'alkylène en C₂-C₃, par exemple l'oxyde d'éthylène ou l'oxyde de propylène ou un mélange d'oxyde d'éthylène et d'oxyde de propylène dans des proportions quelconques. Ces produits de condensation présentent une chaîne alkoxylée au niveau de la fonction hydroxyle du groupe carboxylique. Des tensioactifs préférés de ce groupe sont les produits de condensation obtenus à partir de l'acide oléique, de l'acide palmitique et de l'acide stéarique ;
d) le produit de condensation d'un glycéride d'acide gras en C₈-C₂₂ avec un oxyde d'alkylène en C₂-C₃ tel que l'oxyde d'éthylène et/ou l'oxyde de propylène. Parmi ceux-ci, le palmitate de glycérol éthoxylé est préféré ;
e) le produit de condensation d'un ester d'acide gras en C₈-C₂₂ du sorbitol avec un oxyde d'alkylène en C₂-C₃ qui peut être l'oxyde d'éthylène, l'oxyde de propylène ou leurs mélanges. Ces composés sont des polysorbates. Un exemple préféré est vendu sous la dénomination Tween 80 ;
f) le produit de condensation du sorbitol avec un acide gras en C₈-C₂₂.

On préfère, à titre de tensioactif, utiliser soit un alkylsulfate de métal alcalin soit un tensioactif non ionique du type c) ou du type e) ci-dessus.

Selon l'invention, on peut envisager l'association d'un ou plusieurs tensioactifs.

Ainsi, des associations particulièrement avantageuses de tensioactifs sont l'association :
- d'un tensioactif non ionique du type e) avec un tensioactif non ionique du type f) ;
- d'un tensioactif anionique tel qu'un dialkylsulfosuccinate avec un tensioactif non ionique du type e) ou du type f) ;
- d'un tensioactif anionique tel qu'un alkylsulfate avec un tensioactif non ionique du type e) ou du type f).

De préférence, la composition de l'invention comprend de 8 à 30% en poids de tensioactif, mieux encore de 10 à 30% en poids, par exemple de 10 à 25% en poids.

La composition de l'invention comprend un ou plusieurs alcools en C₁-C₁₂ linéaires ou ramifiés à titre de cotensioactif. De préférence, ces constituants représentent de 5 à 25% en poids de la composition, mieux encore de 10 à 20% en poids.

Les alcools de ce type utilisables selon l'invention sont ceux généralement utilisés dans la technique des microémulsions. On se rapportera par exemple à l'ouvrage de Bourrel M. & Schechter R.S. intitulé "Microemulsions and related systems" Ed. Marcel Dekker Inc., New-York and Basel, 1988.

Des cotensioactifs préférés sont les alcools monohydroxylés aliphatiques et plus particulièrement les alcanols en C₁-C₄, tels que l'isopropanol et l'éthanol.

Les composés hydrosolubles à fonction carbonyle ou hydroxyle ont pour effet de stabiliser la microémulsion de l'invention du point de vue chimique. Ce ne sont pas des tensioactifs. La nature exacte de ces composés est quelconque dès lors qu'ils comprennent au moins une fonction carbonyle ou au moins une fonction hydroxyle, celles-ci pouvant être la fonction carbonyle ou hydroxyle d'un groupe carboxylique.

Il doit être entendu selon l'invention que le composé hydrosoluble peut comprendre à la fois une fonction hydroxyle et une fonction carbonyle.

Un groupe préféré de tels composés hydrosolubles est constitué par les glycols et leurs éthers alkyliques en C₁-C₄ correspondants.

Dans le cadre de l'invention, l'expression glycol désigne tout composé répondant à la formule HO-A-OH dans laquelle A représente une chaîne alkylène éventuellement interrompue par un ou plusieurs atomes d'oxygène, ladite chaîne alkylène étant linéaire ou ramifiée mais de préférence linéaire.

La chaîne alkylène peut comprendre de 2 à 100 atomes de carbone et jusqu'à 50 atomes d'oxygène.

Des exemples de glycols sont l'éthylèneglycol, le propylèneglycol, le diéthylèneglycol et les polyéthylèneglycols.

Les polyéthylèneglycols préférablement utilisés dans le cadre de l'invention ont une masse molaire inférieure ou égale à 2000.

Les monoéthers alkyliques en C₁-C₄ de ces glycols sont également des composés hydrosolubles préférés. Par alkyle, on entend selon l'invention les groupes hydrocarbonés aliphatiques saturés, linéaires ou ramifiés, tels que méthyle, éthyle, propyle, isopropyle, butyle, isobutyle et tert-butyle.

D'autres composés hydrosolubles préférés sont les monosaccharides ou disaccharides du type du sorbitol, du glucose, du galactose, du lactose, du mannitol, du xylitol, du lactol, le lyxose, l'arabinose, l'idose, le mannose, le fructose, le xylose, l'altrose, l'érythrose, le thréose, le fucose, le rhamnose, le ribose et le sorbose.

On peut citer également le glycérol, les monoéthers et les diéthers alkyliques de glycérol dans lesquels les parties alkyle sont en C₁-C₄, la N-méthylpyrrolidone, le N-méthylacétamide, le N,N-diméthylacétamide et l'acétone.

De préférence, la composition comprend un ou plusieurs composés hydrosolubles choisis parmi le N-méthylacétamide, le N,N-diméthylacétamide, la N-méthylpyrrolidone, un glycol éventuellement sous forme de monoéther d'alkyle en C₁-C₄, un monosaccharide, un disaccharide ou le glycérol. Les glycols et leurs éthers ainsi que la N-méthylpyrrolidone sont particulièrement préférés.

La teneur en composés hydrosolubles à fonction carbonyle ou hydroxyle varie entre 5 et 35% en poids de la composition, de préférence entre 10 et 30%, mieux encore entre 15 et 30%.

La microémulsion de l'invention peut comprendre en outre un ou plusieurs composés lipophiles, immiscibles à l'eau, dont la nature est peu critique dès lors que ces composés sont chimiquement inertes vis-à-vis des autres constituants de la composition. Ces composés sont dénués de propriétés tensioactives.

Selon un mode de réalisation préféré, le composé lipophile est choisi parmi les paraffines et isoparaffines, de préférence les hydrocarbures saturés en C₅-C₁₆, les esters d'acide gras en C₄-C₂₀ avec des alcools variés et, par exemple, avec le glycérol ou les (C₄-C₂₀)alkyl-pyrrolidone. Les esters d'acides gras avec le glycérol englobent à la fois les monoglycérides d'acides gras, les diglycérides d'acides gras et les triglycérides d'acides gras.

Un exemple d'alkylpyrrolidone est l'octyl-pyrrolidone.

La présence d'un ou plusieurs constituants lipophiles renforce la stabilité physique de la microémulsion.

Lorsqu'ils sont présents, ces constituants représentent préférablement de 5 à 35% du poids total de la microémulsion, mieux encore de 8 à 20% du poids total de la microémulsion.

Les microémulsions de l'invention peuvent comprendre en outre des agents conservateurs antimicrobiens tels qu'un ester de paraben ou l'alcool benzylique, ou bien des antioxydants choisis parmi les esters de l'acide gallique (tel que le gallate de propyle), l'éthoxyquine, la vitamine E, le butylhydroxyanisole, le butylhydroxytoluène et la diphénylamine.

Les microémulsions de l'invention sont préparées simplement par mélange des constituants dans n'importe quel ordre. Il est préférable cependant de préparer un prémélange à partir des acides gras, des composés immiscibles à l'eau et des composés hydrosolubles, puis d'ajouter à ce prémélange les tensioactifs, les cotensioactifs et l'eau. Selon l'invention, les microémulsions sont du type huile-dans-eau.

Elles comprennent préférablement de 5 à 80% en poids d'eau, mieux encore de 15 à 35% en poids.

La taille des gouttelettes des microémulsions varie généralement de 0,01 à 0,1 µm. Du fait de la petite taille des gouttelettes, les microémulsions sont translucides et non opaques.

Les propriétés avantageuses des microémulsions de l'invention sont notamment :
- une bonne stabilité chimique : les acides gras ne réagissent pas avec les alcools cotensioactifs de la composition ;
- une bonne stabilité physique : les compositions de l'invention restent limpides, sans qu'aucune agglomération des gouttelettes ou séparation de phase ne soit observée ; et
- les microémulsions de l'invention présentent une faible viscosité ; et ceci, pour une large gamme de température, et notamment entre 0 et 40° C.

Les microémulsions de l'invention sont administrables à l'homme et à l'animal par voie orale, sublinguale, intraveineuse, transdermique, intramusculaire, topique, sous-cutanée, rectale ou même par simple mise en contact avec les organes olfactifs situés à l'entrée des voies respiratoires du principe actif de la microémulsion de l'invention, sous forme liquide ou de vapeur.

Ainsi, l'administration peut être réalisée par pulvérisation, nébulisation ou atomisation des microémulsions dans l'environnement de l'homme ou de l'animal ou évaporation du principe actif après application de la microémulsion dans cet environnement, via les mécanismes respiratoires naturels de l'homme ou de l'animal, ou bien encore l'administration peut être réalisée par inhalation.

Le mode d'administration approprié dépend de la nature des constituants de la microémulsion et notamment de la nature des acides gras incorporés à celle-ci.

Lorsque la microémulsion contient des acides gras susceptibles de modifier le comportement ou les réponses physiologiques de l'homme ou de l'animal à titre de principe actif, une administration par simple pulvérisation, nébulisation, atomisation ou application de la composition dans l'environnement de l'homme ou de l'animal est parfaitement appropriée.

Les doses administrées dépendent du mode d'administration, de la nature des principes actifs, de la gravité de l'affection ou pathologie et du poids de l'animal et/ou de la nature du comportement ou de la réponse physiologique visé.

Ainsi, selon un autre de ses aspects, l'invention concerne l'utilisation de compositions de l'invention, en vue de modifier le comportement de l'homme ou de l'animal sans qu'un traitement thérapeutique ou prophylactique ne soit impliqué.

Selon encore un autre de ses aspects, l'invention concerne l'utilisation des microémulsions de l'invention pour la préparation d'un médicament administrable à l'homme ou à l'animal.

A titre d'exemple, les microémulsions à base d'un des mélanges d'acides gras décrits dans US 5,709,863 peuvent être utilisées dans le traitement de l'anxiété chez le chat ou plus généralement en vue de modifier les réponses physiologiques de l'homme ou de l'animal.

Les exemples qui suivent illustrent plus avant l'invention.

### EXEMPLE 1

On prépare les compositions suivantes dont la formulation est rapportée dans les tableaux 1 et 2 suivants, par simple mélange des constituants:

**TABLEAU 1**

| Constituants | Formulation 1.1 (parties en poids) | Formulation 1.2 (parties en poids) |
|---|---|---|
| - Acide oléique | 3,00 | 4,20 |
| - Acide linoléique | 4,20 | 5,25 |
| - Acide palmitique | 2,80 | 3,75 |
| - Acide palmitoléique | - | 1,80 |
| - Ether diéthylique de diéthylèneglycol | - | 20,00 |
| - N-méthylpyrrolidone | 20,00 | - |
| - Oléate de PEG 600 | - | 15,00 |
| - Laurylsulfate de sodium | 10,00 | - |
| - Isopropanol | 10,00 | 15,00 |
| - Eau | 50,00 | 35,00 |

**TABLEAU 2**

| Constituants | Formulation 1.3 (parties en poids) | Formulation 1.4 (parties en poids) |
|---|---|---|
| - Acides gras | 20,00 | 20,00 |
| - Isopropanol | 10,00 | 20,00 |
| - N-méthylpyrrolidone | 24,75 | 25,70 |
| - Polysorbate 80 | 25,00 | 25,00 |
| - Agents conservateurs | - | 1,01 |
| - Eau | 20,25 | 8,29 |

Les formulations 1.3 et 1.4 contiennent plus précisément les proportions suivantes d'acides gras en C₁₀-C₁₈ :

| Acides gras | Formulation 1.3 | Formulation 1.4 |
|---|---|---|
| Acide linoléique | 7,0 | 7,0 |
| Acide oléique | 6,2 | 6,2 |
| Acide palmitique | 4,0 | 4,0 |
| Acide myristique | 1,2 | 1,2 |
| Acide laurique | 1,2 | 1,2 |
| Acide caprique | 0,4 | 0,4 |

### EXEMPLE 2

Dans cet exemple, on a étudié la stabilité des compositions de formulation 1.3 et 1.4 de l'exemple 1.

Les compositions testées ont été maintenues 6 mois à 25° C.

Un autre test a été réalisé en maintenant les microémulsions 3 à 6 mois à 40° C.

Pendant toute la durée des tests, les formulations 1.3 et 1.4 sont restées limpides.

Une analyse de la teneur respective en acides gras après 6 mois montre par ailleurs une excellente stabilité des compositions à 25° C.

A 40° C, on observe après 6 mois une légère modification de la formulation en acides gras dans le cas de la composition 1.3 :
- la teneur en acide linoléique est réduite de 6% en poids ;
- la teneur en acide palmitique est réduite de 2% en poids ; et
- la teneur en acide myristique est réduite de 1% en poids.

A 40° C, on observe après 3,5 mois une légère modification de la formulation en acides gras dans le cas de la composition 1.4 :
- la teneur en acide linoléique est réduite de 2% en poids ;
- la teneur en acide palmitique est réduite de 3% en poids ;
- la teneur en acide myristique est réduite de 5% en poids.

Ces résultats confirment l'excellente stabilité physique et chimique des microémulsions de l'invention.

## Revendications

1. Composition non détergente à base d'acides gras, stable, sous forme de microémulsion, pour l'administration à l'homme ou à l'animal, comprenant :
- de 5 à 30% en poids d'un ou plusieurs acides gras à fonction acide carboxylique libre, à titre de principe actif ;
- de 5 à 35% en poids d'un ou plusieurs tensioactifs ;
- de 5 à 30% en poids d'un ou plusieurs alcools en C₁-C₁₂, à titre de cotensioactif ;
- de 5 à 35% en poids d'un ou plusieurs composés hydrosolubles à fonction carbonyle ou hydroxyle ; et
- de 0 à 35% en poids d'un ou plusieurs composés immiscibles à l'eau ;
ladite composition présentant un pH inférieur à 6,5.

2. Composition selon la revendication 1, **caractérisée en ce que** le principe actif est constitué d'un ou plusieurs acides gras en C₄-C₂₂ à fonction acide carboxylique libre.

3. Composition selon l'une quelconque des revendications 1 ou 2, **caractérisée en ce que** le principe actif est constitué d'un ou plusieurs acides gras susceptibles de modifier le comportement ou les réponses physiologiques de l'homme ou de l'animal.

4. Composition selon l'une quelconque des revendications 1 ou 2, **caractérisée en ce que** le principe actif est une phéromone constituée d'un ou plusieurs acides gras.

5. Composition selon l'une quelconque des revendications 1 et 2, **caractérisée en ce que** le principe actif comprend de l'acide oléique et de l'acide palmitique.

6. Composition selon l'une quelconque des revendications 1 et 2, **caractérisée en ce que** le principe actif comprend de l'acide oléique et de l'acide n-butyrique.

7. Composition selon l'une quelconque des revendications 1 et 2, **caractérisée en ce que** le principe actif comprend de l'acide palmitique, de l'acide oléique et de l'acide linoléique.

8. Composition selon l'une quelconque des revendications 1 et 2, **caractérisée en ce que** le principe actif comprend de l'acide palmitique, de l'acide oléique, de l'acide linoléique et de l'acide myristique.

9. Composition selon l'une quelconque des revendications 1 et 2, **caractérisée en ce que** le principe actif comprend de l'acide palmitique, de l'acide oléique, de l'acide linoléique et de l'acide palmitoléique.

10. Composition selon l'une quelconque des revendications précédentes, **caractérisée en ce qu'**elle comprend un ou plusieurs tensioactifs anioniques ou non ioniques.

11. Composition selon la revendication 10, **caractérisée en ce qu'**elle comprend à titre de tensioactif un (C₆-C₁₈)alkylsulfate de métal alcalin ; le produit de condensation d'un acide gras en C₈-C₂₂ avec un oxyde d'alkylène en C₂-C₃ ; ou bien le produit de condensation d'un ester d'acide gras en C₈-C₂₂ du sorbitol avec un oxyde d'alkylène en C₂-C₃.

12. Composition selon l'une quelconque des revendications précédentes, **caractérisée en ce que** ledit cotensioactif est un ou plusieurs alcanols en C₁-C₄, de préférence l'alcool éthylique ou l'alcool isopropylique.

13. Composition selon l'une quelconque des revendications précédentes, **caractérisée en ce qu'**elle comprend un ou plusieurs composés hydrosolubles choisis parmi le N-méthylacétamide, le N,N-diméthylacétamide, la N-méthylpyrrolidone, un glycol éventuellement sous forme de monoéther d'alkyle en C₁-C₄, un monosaccharide, un disaccharide ou le glycérol.

14. Composition selon l'une quelconque des revendications précédentes, **caractérisée en ce qu'**elle comprend un ou plusieurs composés immiscibles à l'eau choisis parmi un hydrocarbure saturé en C₅-C₁₆, un mono-, di, ou triglycéride d'acide gras en C₄-C₂₀ ou une (C₄-C₂₀)alkyl-pyrrolidone.

15. Composition selon l'une quelconque des revendications précédentes, **caractérisée en ce que** son pH est compris entre 3 et 6.

16. Composition selon l'une quelconque des revendications précédentes, **caractérisée en ce qu'**elle comprend :
- de 10 à 30% en poids d'un ou plusieurs acides gras à fonction acide carboxylique libre ;
- de 10 à 30% en poids d'un ou plusieurs tensioactifs non ioniques ou anioniques ;
- de 5 à 25% en poids d'un ou plusieurs alcools en C₁-C₁₂ ; et
- de 10 à 30% en poids d'un ou plusieurs composés hydrosolubles.

17. Utilisation d'une composition selon l'une quelconque des revendications 3 à 16 pour l'administration à l'homme ou à l'animal en vue de modifier le comportement de l'homme ou de l'animal, sans qu'un traitement thérapeutique ou prophylactique ne soit impliqué.

18. Utilisation d'une composition selon l'une quelconque des revendications 1 à 16 pour la préparation d'un médicament administrable à l'homme ou à l'animal.

19. Utilisation selon la revendication 18, en vue de modifier les réponses physiologiques de l'homme ou de l'animal.

20. Utilisation selon l'une des revendications 17 ou 19, **caractérisée en ce que** l'administration est réalisée par mise en contact avec les organes olfactifs situés à l'entrée des voies respiratoires de l'homme ou de l'animal du principe actif contenu dans ladite composition.

21. Utilisation selon la revendication 20, **caractérisée en ce que** la mise en contact a lieu via les mécanismes respiratoires naturels de l'homme ou de l'animal après pulvérisation, nébulisation ou atomisation de ladite microémulsion dans l'environnement de l'homme ou de l'animal ou après application de ladite microémulsion dans cet environnement.

## Claims

1. Stable non-detergent composition based on fatty acids, in the form of a microemulsion, for administration to humans or animals, comprising:
- 5 to 30 % by weight of one or more fatty acids with a free carboxylic acid function, as active ingredient;
- 5 to 35 % by weight of one or more surfactants;
- 5 to 30 % by weight of one or more C₁-C₁₂ alcohols as co-surfactant;
- 5 to 35 % by weight of one or more water-soluble compounds with a carbonyl or hydroxyl function; and
- 0 to 35 % by weight of one or more water-immiscible compounds;
said composition having a pH of less than 6.5.

2. Composition according to claim 1, **characterised in that** the active ingredient is made up of one or more C₄-C₂₂ fatty acids with a free carboxylic acid function.

3. Composition according to either of claims 1 and 2, **characterised in that** the active ingredient is made up of one or more fatty acids capable of modifying behaviour or physiological responses in humans or animals.

4. Composition according to either of claims 1 and 2, **characterised in that** the active ingredient is a pheromone consisting of one or more fatty acids.

5. Composition according to either of claims 1 and 2, **characterised in that** the active ingredient comprises oleic acid and palmitic acid.

6. Composition according to either of claims 1 and 2, **characterised in that** the active ingredient comprises oleic acid and n-butyric acid.

7. Composition according to either of claims 1 and 2, **characterised in that** the active ingredient comprises palmitic acid, oleic acid and linoleic acid.

8. Composition according to either of claims 1 and 2, **characterised in that** the active ingredient comprises palmitic acid, oleic acid, linoleic acid and myristic acid.

9. Composition according to either of claims 1 and 2, **characterised in that** the active ingredient comprises palmitic acid, oleic acid, linoleic acid and palmitoleic acid.

10. Composition according to any one of the preceding claims, **characterised in that** it comprises one or more anionic or non-ionic surfactants.

11. Composition according to claim 10, **characterised in that** it contains as surfactant an alkali metal (C₆-C₁₈)alkyl sulphate; the condensation product of a C₈-C₂₂ fatty acid with a C₂-C₃ alkylene oxide; or the condensation product of a sorbitol C₈-C₂₂ fatty acid ester with a C₂-C₃ alkylene oxide.

12. Composition according to any one of the preceding claims, **characterised in that** said co-surfactant is one or more C₁-C₄ alkanols, preferably ethyl alcohol or isopropyl alcohol.

13. Composition according to any one of the preceding claims, **characterised in that** it comprises one or more water-soluble compounds selected from among N-methyl acetamide, N,N-dimethyl acetamide, N-methyl pyrrolidone, a glycol optionally in the form of a C₁-C₄ alkyl monoether, a monosaccharide, a disaccharide or glycerol.

14. Composition according to any one of the preceding claims, **characterised in that** it comprises one or more water-immiscible compounds selected from among a C₅-C₁₆ saturated hydrocarbon, a C₄-C₂₀ fatty acid mono-, di- or triglyceride or a (C₄-C₂₀)alkyl-pyrrolidone.

15. Composition according to any one of the preceding claims, **characterised in that** its pH is between 3 and 6.

16. Composition according to any one of the preceding claims, **characterised in that** it comprises:
- 10 to 30 % by weight of one or more fatty acids with a free carboxylic acid function;
- 10 to 30 % by weiqht of one or more non-ionic or anionic surfactants;
- 5 to 25 % by weight of one or more C₁-C₁₂ alcohols; and
- 10 to 30 % by weight of one or more water-soluble compounds.

17. Use of a composition according to any one of claims 3 to 16 for administration to humans or animals with the aim of modifying the behaviour of the humans or animals, without implying any therapeutic or prophylactic treatment.

18. Use of a composition according to any one of claims 1 to 16 for preparing a medicament which can be administered to humans or animals.

19. Use according to claim 18, with the aim of modifying physiological responses in humans or animals.

20. Use according to one of claims 17 or 19, **characterised in that** administration is effected by bringing the active ingredient contained in said composition into contact with the olfactory organs situated at the entrance to the respiratory tract in the human or animal.

21. Use according to claim 20, **characterised in that** the contact takes place through the natural respiratory mechanisms of the human or animal after spraying, nebulising or atomising said microemulsion in the environment of the human or animal or after said microemulsion has been applied to said environment.

## Patentansprüche

1. Stabile Nicht-Detergenz-Zusammensetzung auf Basis von Fettsäuren in Form einer Mikroemulsion zur Verabreichung an einen Menschen oder ein Tier, die umfasst:
- 5 bis 30 Gew.-% einer oder mehrerer Fettsäuren mit freien Carbonsäuregruppen als aktiven Wirkstoff,
- 5 bis 35 Gew.-% eines oder mehrerer Tenside,
- 5 bis 30 Gew.-% eines oder mehrerer C₁-C₁₂-Alkokole als Co-Tensid,
- 5 bis 35 Gew.-% einer oder mehrerer wasserlöslicher Verbindungen mit Carbonyl- oder Hydroxylgruppen, und
- 0 bis 35 Gew.-% einer oder mehrerer mit Wasser nicht mischbaren Verbindungen;
wobei die Zusammensetzung einen pH niedriger als 6,5 aufweist.

2. Zusammensetzung gemäß Anspruch 1, **dadurch gekennzeichnet, dass** der aktive Wirkstoff aus einer oder mehreren C₄-C₂₂-Fettsäuren mit freien Carbonsäuregruppen besteht.

3. Zusammensetzung gemäß einem der Ansprüche 1 oder 2, **dadurch gekennzeichnet, dass** der aktive Wirkstoff aus einer oder mehreren Fettsäuren besteht, die in der Lage sind, das Verhalten oder die physiologischen Reaktionen eines Menschen oder eines Tieres zu verändern.

4. Zusammensetzung gemäß einem der Ansprüche 1 oder 2, **dadurch gekennzeichnet, dass** der aktive Wirkstoff ein Pheromon ist, das aus einer oder mehreren Fettsäuren besteht.

5. Zusammensetzung gemäß einem der Ansprüche 1 oder 2, **dadurch gekennzeichnet, dass** der aktive Wirkstoff Oleinsäure und Palmitinsäure umfasst.

6. Zusammensetzung gemäß einem der Ansprüche 1 oder 2, **dadurch gekennzeichnet, dass** der aktive Wirkstoff Oleinsäure und N-Butyrsäure umfasst.

7. Zusammensetzung gemäß einem der Ansprüche 1 oder 2, **dadurch gekennzeichnet, dass** der aktive Wirkstoff Palmitinsäure, Oleinsäure und Linolsäure umfasst.

8. Zusammensetzung gemäß einem der Ansprüche 1 oder 2, **dadurch gekennzeichnet, dass** der aktive Wirkstoff Palmitinsäure, Oleinsäure, Linolsäure und Myristinsäure umfasst.

9. Zusammensetzung gemäß einem der Ansprüche 1 oder 2, **dadurch gekennzeichnet, dass** der aktive Wirkstoff Palmitinsäure, Oleinsäure, Linolsäure und Palmitoleinsäure umfasst.

10. Zusammensetzung gemäß einem der vorhergehenden Ansprüche, **dadurch gekennzeichnet, dass** sie eines oder mehrere anionische oder nicht-ionische Tenside umfasst.

11. Zusammensetzung gemäß Anspruch 10, **dadurch gekennzeichnet, dass** sie als Tensid ein (C₆-C₁₈)-Alkylsulfat eines Alkalimetalls, das Kondensationsprodukt einer C₈-C₂₂ Fettsäure mit einem C₂-C₃-Alklylenoxids, oder auch das Kondensationsprodukt einer C₈-C₂₂-Fettsäure des Sortibols mit einem C₂-C₃ Alklylenoxids umfasst.

12. Zusammensetzung gemäß einem der vorhergehenden Ansprüche, **dadurch gekennzeichnet, dass** das Co-Tensid ein oder mehrere C₁-C₄ Alkanole ist, vorzugsweise des Ethylalkohols oder Isopropylalkohols.

13. Zusammensetzung gemäß einem der vorhergehenden Ansprüche, **dadurch gekennzeichnet, dass** sie eine oder mehrere wasserlösliche Verbindungen umfasst, die aus N-Methylacetamid, N,N-Dimethlyacetamid, N-Methylpyrrolidon, einem Glykol, gegebenenfalls in Form eines C₁-C₄-Alkylmonoethers, einem Disaccharid oder Glycerin ausgewählt werden.

14. Zusammensetzung gemäß einem der vorhergehenden Ansprüche, **dadurch gekennzeichnet, dass** die eine oder mehrere mit Wasser nicht mischbare Verbindungen umfasst, die aus gesättigten C₅-C₁₆-Kohlenwasserstoffen, einem Mono-, Di- oder Triglycerid einer C₄-C₂₀-Fettsäure oder einem (C₄-C₂₀)-Alkylpryrrolidon ausgewählt werden.

15. Zusammensetzung gemäß einem der vorhergehenden Ansprüche, **dadurch gekennzeichnet, dass** ihr pH zwischen 3 und 6 liegt.

16. Zusammensetzung gemäß einem der vorhergehenden Ansprüche, **dadurch gekennzeichnet, dass** sie umfasst:
- 10 bis 30 Gew.-% einer oder mehrerer Fettsäuren mit freien Carbonsäuregruppen,
- 10 bis 30 Gew.-% eines oder mehrerer anionischer oder nicht-ionischer Tenside,
- 5 bis 25 Gew.-% eines oder mehrerer C₁-C₁₂-Alkokole, und
- 10 bis 30 Gew.-% einer oder mehrerer wasserlösliche Verbindungen.

17. Verwendung einer Zusammensetzung gemäß einem der Ansprüche 3 bis 16 zur Verabreichung an einen Menschen oder ein Tier mit dem Ziel, das Verhalten des Menschen oder des Tiers zu verändern, ohne dass damit eine therapeutische oder prophylaktische Behandlung verbunden wäre.

18. Verwendung einer Zusammensetzung gemäß einem der Ansprüche 1 bis 16 zur Herstellung eines an einen Menschen oder ein Tier verabreichbaren Medikaments.

19. Verwendung gemäß Anspruch 18 mit dem Ziel, das Verhalten des Menschen oder des Tiers zu verändern.

20. Verwendung gemäß einem der Ansprüche 17 oder 19, **dadurch gekennzeichnet, dass** sie durch In-Kontakt-Bringen der olfaktorischen Organe, die sich am Eingang des Respirationstraktes des Menschen der des Tiers befinden, mit dem aktiven Wirkstoff, der in der Zusammensetzung enthalten ist, durchgeführt wird.

21. Verwendung gemäß Anspruch 20, **dadurch gekennzeichnet, dass** das In-Kontakt-Bringen am Ort über die natürlichen Atmungsmechanismen des Menschen oder des Tieres nach Pulverisierung, Vernebelung oder Zerstäubung der Mikroemulsion in der Umgebung des Menschen oder des Tiers oder nach Anwendung der Mikroemulsion in dieser Umgebung durchgeführt wird.
